(19) Europäisches Patentamt
European Patent Office
Office européen des brevets

(11) **EP 3 375 359 B1**

(12) **EUROPEAN PATENT SPECIFICATION**

(45) Date of publication and mention
of the grant of the patent:
**29.05.2024  Bulletin 2024/22**

(21) Application number: **17161116.3**

(22) Date of filing: **15.03.2017**

(51) International Patent Classification (IPC):
**A61B 5/021** *(2006.01)*      **A61B 7/04** *(2006.01)*
**A61B 5/022** *(2006.01)*      A61B 5/00 *(2006.01)*

(52) Cooperative Patent Classification (CPC):
**A61B 5/02125; A61B 5/02233; A61B 7/04;**
A61B 5/318; A61B 5/6823

(54) **METHOD FOR ANALYSING CARDIOVASCULAR PARAMETERS OF AN INDIVIDUAL**

VERFAHREN ZUR ANALYSE VON KARDIOVASKULÄREN PARAMETERN EINES INDIVIDUUMS

PROCÉDÉ POUR ANALYSER LES PARAMÈTRES CARDIO-VASCULAIRES D'UN INDIVIDU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.09.2018  Bulletin 2018/38**

(73) Proprietor: **Withings
92130 Issy-les-Moulineaux (FR)**

(72) Inventors:
• **MERLOT, Antoine
75005 Paris (FR)**
• **CAMPO, David
92100 Boulogne Billancourt (FR)**
• **VISSAC, Virginie
92100 Boulogne  Billancourt (FR)**

• **BALTI, Haïkel
94400 Vitry sur Seine (FR)**

(74) Representative: **Withings IP
2, rue Maurice Hartmann
92130 Issy-les-Moulineaux (FR)**

(56) References cited:
**US-A1- 2003 220 577    US-A1- 2015 374 244**
**US-A1- 2016 287 172**

Remarks:
The file contains technical information submitted after
the application was filed and not included in this
specification

## Description

### FIELD OF THE DISCLOSURE

The present disclosure relates to methods for analysing cardiovascular parameters of an individual and devices used to carry out such methods.

More particularly, it relates to a method designed to assess parameters of the type "pulse transit time", these parameters being determined from acoustic signals and/or electrical signals emitted by the heart of such individual, and from responsive signals sensed at a blood circulation sensor, which may be a blood pressure sensor or other type of sensor. Likewise for one instance this kind of method, there may be used an inflatable bladder comprised in a blood pressure cuff.

### BACKGROUND OF THE DISCLOSURE

There is known devices that combine arterial pressure sensing means and electrocardiogram sensing means, of the type for example disclosed in document US20160235325, in which some attempts have been done to assess pulse transit time from ECG signal. However, such method shows inaccuracy shortcomings since ECG signal fails to accurately reflect the mechanical activity of the heart. Furthermore, acquiring pressure waves at the wrist is not fully adequate to accurately detect some trouble located in the arterial network close to the heart. Further, US 2003/220577 discloses methods and systems for determining cardiovascular parameters of a patient, including placing a phonocardiogram sensor on a patient's body, and calculating a blood pressure pulse transit time value.

Therefore, the inventors have identified a need to enhance functionalities provided by such methods and improve their accuracy.

### SUMMARY OF THE DISCLOSURE

According to one aspect of the present disclosure, there is disclosed a method according to claim 1.

Thereby, the electronic controller acquires acoustic signals, sensed by the acoustic sensor, in timely conjunction with blood circulation parameters at the predefined position, to determine therefrom for example a Pulse Transit Time (PTT) from the aortic valve to the artery location of interest.

The term "predefined position" should be understood as a predefined location at an artery vessel where the heart pulses reflect. The artery location of interest is not necessarily at the vicinity of the skin, it can be located in depth into a user's limb.

It should be understood that the so-called predefined position can be located at the left arm, but it could also be somewhere else on the left arm, e.g. on the forearm, on the wrist, somewhere else on the right arm of the user, including the wrist; it is not excluded to implement the proposed method with pressure signals taken on a lower limb of the user. Of course, calculation parameters to be used are to be adapted in particular to the length of the arterial blood path (P) for each case.

The inventors have found that acoustic signals provide a good time marker for start time in order to perform calculation of Pulse Transit Time (PTT). Particularly, the closure of the aortic valve produces a sufficient noise to be properly captured.

The term "blood circulation parameters" can designate the blood local pressure, the blood local speed, or even an image of both pressure and speed, prevailing at the predefined position.

In one implementation, the system may comprise means for exerting pressure around a limb of the user, at the predefined position, and the blood sensor may be a pressure sensor, wherein blood fluid circulation parameters signals are pressure signals,
and wherein the method comprises, prior to step /S1/ :
/S0/ - exert a predetermined pressure (PT1)

And at step /S2/ the characteristic point (M2) is determined from a pressure signal curve.

With pressure signals, the method works well even though the artery vessel is deep below the *skin.*

In one implementation, the means for exerting pressure is a band (2) having an inflatable bladder (53) configured to be placed at the predefined position, a pneumatic unit with at least a pump (7), the pressure sensor being configured to be fluidly connected to the inflatable bladder.

While aortic valve closure can be taken as a first time marker and, further, the corresponding reflection/response in the pressure signal at bladder can be taken as a relevant second time marker.

In one implementation, the predefined position is at the left arm (BG) of the user, the band (2) is an arm band and is configured to be placed around the left arm of the user.

It should be noted that, in the case of the left arm, the blood path at interest for assessing the Pulse Transit Time (PTT) is rather short namely less than 40 cm and concentrates on the main arterial network starting from the heart, which enables to detect potential problems affecting aorta or other portion of the main arterial network leading from the heart to the arm, (under this perspective the arm can be a better location than the wrist or a leg).

Thereby, the electronic controller acquires pressure wave signals to determine therefrom for example a Pulse Transit Time (PTT) from the aortic valve to the brachial artery.

According to one particular option, there is disclosed a method to collect cardiovascular data relating to an individual user (U), in a system comprising an acoustic sensor (4) configured to be coupled to the chest of the user, an arm band (2) having an inflatable bladder (53) configured to be placed at a predefined position around a limb of the user, for example at the left arm (BG), a pneumatic unit with at least a pump (7) and a pressure sensor (61), configured to inflate and deflate the inflatable bladder, an arterial blood path (P) being defined from the heart to the predefined position, for example the left arm (BG) of the user, the method comprising a set of steps named PTT procedure :

A1- acquiring acoustic signals at the acoustic sensor,
A2- acquiring pressure signals at the pressure sensor, image of pressure prevailing at the brachial artery,
/S0/ - inflate the bladder at a predetermined pressure (PT1)
/S1/- determining aortic valve closing instant T1(k) from acoustic signals,
/S2/- determining subsequently, from pressure signals, a characteristic point (M2) of the pressure signal curve occurring at instant T2(k),
/S3/- calculate a time difference, defined as $\Delta T(k) = T2(k) - T1(k)$
/Sloop/ repeating, for each heartbeat, steps S1 to S3 until a stop criterion (SC) is met.

The arterial blood path of interest is therefore mainly focused on the central arterial distribution network; this improves the detection of potential problems affecting aorta or other portion of the main arterial network leading from the heart to the left arm.

The blood path at interest for assessing the Pulse Transit Time (PTT) is rather short namely less than 40 cm and concentrates on the main arterial network starting from the heart, which enables to detect potential problems affecting aorta or other portion of the main arterial network leading from the heart to the left arm.

According to one particular option, the characteristic point (M2) of the pressure signal curve is defined as a succession of a maximum apex (M1) and a minimum apex (M2), said instant T2(k) being defined as the instant when said minimum apex occurs.

Faithfull and relevant time marker corresponding to arrival at the arm of the effect of the closure of aortic valve is thus determined.

According to one particular option, the system comprises a set of contact electrodes (3) for electrocardiographic sensing, configured to be brought in contact with the skin of the user U, the method comprising the steps:

/A3/- acquiring ECG signals at the contact electrodes,
/S10/ - determining a characteristic QRS signal from ECG signals as a synchronization signal (T0) reflecting heartbeat.

This helps to synchronize the analysis of acoustic and blood circulation signals.

According to one particular option, the aortic valve closing instant T1(k) is defined as a second significant sound (B2) of the heartbeat.

Faithfull/relevant time marker corresponding to the closure of aortic valve is thus determined.

According to one particular option, wherein at step /S1/, the aortic valve closing instant T1(k) is determined as follows :

/S11/- identifying a first significant sound (B1) of the heartbeat, reflecting mitral valve closing, just following QRS signal at instant T0,
/S12/- identifying a second significant sound (B2) of the heartbeat, reflecting aortic valve closing, and record said second significant sound (B2) as instant T1(k).

This provides a simple and reliable method to determine instant T1(k).

According to one particular option, a significant sound is defined whenever a instantaneous power of the acoustic signals exceeds a predetermined threshold (BS).

Noises can thus be disregarded.

According to one particular option, the method comprises, before the PTT procedure, a preliminary set of steps named Blood Pressure procedure comprising the steps :

/Ph1/- start inflating the bladder,
/Ph2/- stop inflating the bladder (when no more pressure wave is identified),
/Ph3/- start deflating the bladder,
meanwhile are performed the following steps :

/PhS/ - determining Systolic Blood pressure (PTS), during inflating phase and/or deflating phase
/PhD/ - determining Diastolic Blood pressure (PTD), during inflating phase and/or deflating phase

This provides a blood pressure reference prior to carry out PTT procedure; abortion of PTT can be decided if BP procedure is incorrect.

According to one particular option, prior to steps /S1/ to /S3/, the predetermined pressure **PT1,** providing pressurization for PTT procedure, is calculated with reference to the diastolic pressure (PTD) determined at step PhD.

PT1 is therefore user dependent.

According to one particular option, the predetermined pressure **PT1** can be defined as a function of **PTD,** for example with a value below PTD, this difference defined by a predefined offset; in other words, PT1 can be equal to PTD - PTof, with PTof for example equal to 10 mmHg (10 Torr).

According to one particular option, the stop criterion SC is met after N heartbeats for which steps S1 to S3 were carried out properly, N being comprised between 4 and 10.

PTT procedure can be short. Time duration can be defined by user or according to a quality index criteria. Respiration/Breathing or other disturbances can be compensated for. According to one particular option, at steps /A1/ and/or /A2/, acoustic signals and/or pressure signals are digitalized are further filtered with a band pass filter having a passing band range of [0,5Hz - 1kHz].

Continuous components and noises can thus be eliminated.

According to one particular option, during PTT procedure, pump is not energized and a bleeder valve comprised in the pneumatic unit is not energized.

No noise from the device itself : no disturbance on acoustic signals.

According to one particular option, the method may further comprise :

/S41/ - calculate an average value $\Delta\textbf{Tav}$ of $\Delta\textbf{T(k)}$, for **k=j to j+N** (with N being the number of heartbeats with effective PTT measurement),

/S42/- calculate a Pulse Wave Velocity (PWV) defined as **PWV(k) = length(P) / $\Delta$Tav,**

/S5/- assess therefrom an arterial stiffness (AS) of the user.

This provides a reliable rating averaged over several cycles.

According to one particular option, the **height** (UH) of the user is taken into account at step /S42/, namely **length(P) = F1 (UH)**

This provides a personal index, which is relevant whatever the length of the blood path (which depends on the height of the user at first order).

According to one particular option, age (UA), gender (UG), and weight (UW), of the user is additionally taken into account at step /S42/, namely **length(P) = F2 (UH,UA,UG,UW).**

A cardiovascular index can be refined to faithfully reflect the arterial stiffness, irrespective of user profile particulars.

According to one particular option, subsequent evaluations of Pulse Wave Velocity are recorded for a particular user to form a history curve, and a deviation in said curve is notified to said particular user.

The change in user habits can be detected by this means; said change can relate to food intake, physical exercise, initial phase of a disease etc.

The present disclosure is also directed to a <u>system</u>, <u>device</u> or <u>apparatus</u> configured to carry out the above-mentioned methods and functionalities.

The present disclosure is also directed to computer readable medium encoded with instructions that, when executed by a computer, cause performance of a method as described above.

BRIEF DESCRIPTION OF THE DRAWINGS

Other features and advantages of the disclosure appear from the following detailed description of one of its embodiments, given by way of non-limiting example, and with reference to the accompanying drawings, in which:

- Figure 1 illustrates a general overview of a device according to the present disclosure in a use configuration,
- Figure 2 shows a diagrammatic sectional view of the device in place on the left arm of the user, and adjacent to the chest, for the generically defined embodiment,
- Figure 3 illustrates a mechanical configuration of the device in an open configuration, according to a first embodiment,
- Figure 4 shows a diagrammatic sectional view of the device in place on the left arm of the user, according to the embodiment shown in Figure 3,
- Figure 5 shows another diagrammatic sectional view of the device in place on the left arm of the user, according to a second embodiment,
- Figure 6 illustrates a buckle configured to be used in co-operation with the arm band, according to the second embodiment
- Figure 7 shows an exemplary block diagram of the device,
- Figure 8 shows a timing chart illustrating the method, at the heartbeat timescale,
- Figure 9 shows a timing chart illustrating the method at a larger timescale,
- Figure 10 illustrates the mechanical configuration of the device of the first embodiment in a stowed configuration.
- Figure 11 illustrates a general perspective view of the device of the first embodiment,
- Figure 12 illustrates a general overview of the disclosed method,
- Figure 13 shows steps of the method regarding pressure signals handling,
- Figure 14 shows steps of the method regarding pulse transit time handling,
- Figure 15 shows a detailed sectional view of the armband.

DETAILLED DESCRIPTION OF THE DISCLOSURE

In the figures, the same references denote identical or similar elements. For clarity purposes, some parts are represented intentionally not at scale with regard to other parts. Also, some parts of timing charts can be represented intentionally not at scale.

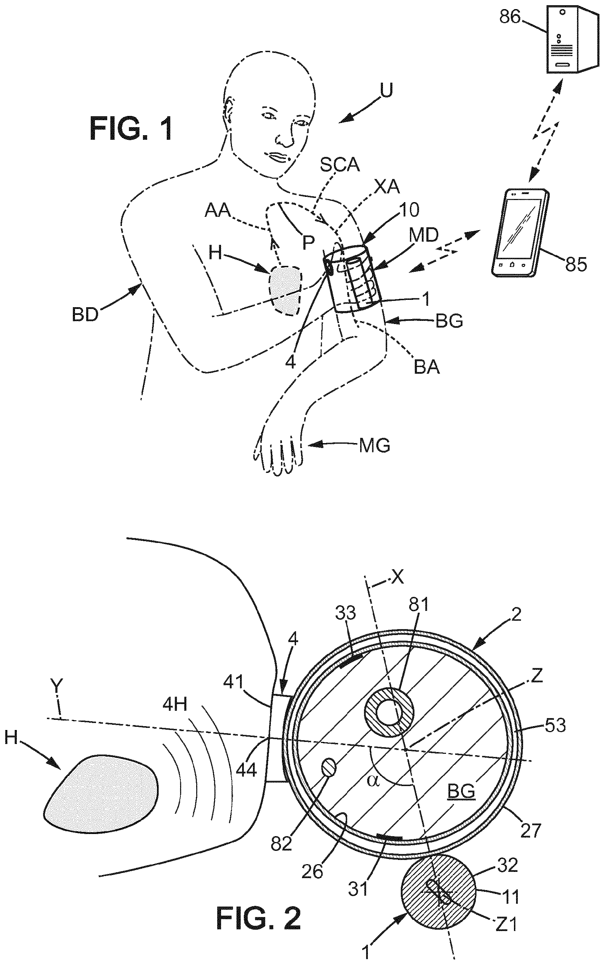

Figure 1 shows an individual (also 'user') **U** in a configuration where he/she is using a device **10** according to the present disclosure. The device (otherwise called "apparatus") **10** looks like a known brachial blood pressure sensing device (commonly named Blood Pressure Monitor i.e. in short "BP Monitor"), but the device exhibits extended functionalities as will be apparent below, so that the device can be called 'upgraded BP Monitor.

The user **U** in question has, among other organs and limbs in his/her anatomy : a left arm **BG,** a right arm **BD,** a heart **H,** a left hand **MG** a right hand **MD.**

Further the user in question has an aortic artery ('aortic arch') denoted **AA,** a subclavian artery **SCA,** an axillary artery **XA,** a brachial artery **BA,** belonging generally to the cardiovascular system of the user. Therefore a blood path of interest noted **P** is defined as the fluid conduit from the heart **H** to a reference point at the brachial artery **BA.**

Besides the left arm brachial artery as reference point, it should be understood that the proposed method would properly work with another predefined position where a band can be secured around a limb, either a lower limb or an upper limb.

The device **10** has a wireless communication capability to exchange data with a mobile entity like a smartphone **85** (more generally a mobile device belonging to the user U like a tablet, a laptop....). Such smartphone **85** may in turn exchange data with a remote entity like an Internet server **86** (more generally any resource available somewhere in Internet, not excluding a so-called "cloud" resource).

The device **10** has either a small display or no display at all, since the user interface capability provided by the smartphone **85** is fully relevant to support displays relating to the use and extended functionalities of the device.

The device **10** is intended to be used at a home environment, for healthy people as well as people suffering from some disease. It may be used in a medical environment but is particularly suitable to be used by non-medical personnel, i.e. the user under measurement him/herself.

In the shown example, the device **10** comprises an armband **2** wrapped around the left arm **BG,** a control unit assembly **1,** and an acoustic sensor denoted **4.**

The rest of the time the device is stowed, notably in a folded configuration as will be seen later.

As illustrated on Figure 1, the device comprises an armband wrapped around the arm i.e. the part of the upper limb comprised between the shoulder and the elbow. However it is not excluded to use the device elsewhere, at the forearm for example.

As illustrated on Figure 1, the device is installed on the left arm of the user. However it is not excluded to use the device elsewhere, at the right arm for example.

As illustrated on Figure 2, the left arm of the user includes a bone named humerus denoted **81,** muscles (not specially shown), and the abovementioned brachial artery denoted **82.** The humerus extends along an axis denoted **Z.** The armband band **2,** when wrapped around the arm BG, has a general cylindrical shape with a reference axis substantially coinciding with arm axis **Z.**

The armband has an internal wall denoted **26** intended to contact the arm's skin and to press against the arm. The armband has an external wall denoted **27** on the opposite side of the band with regard to the internal wall **26.**

In use configuration, the acoustic sensor **4** is located against the chest, i.e. against the left side of chest. Sound waves **4H** emitted by the heart are sensed by a sensitive portion **41** of the acoustic sensor **4,** the sensitive portion 41 bearing on the left-side chest, i.e. adjacent to the chest. Handling of electrical signals transduced from acoustic waves **4H** will be detailed later. It should be noted that acoustic waves 4H can be sensed by the sensitive portion **41** without trouble through a light clothing, an underwear or the like.

According to one particular option, the device is further equipped with an ECG function, i.e. ElectroCardioGraphic function.

For this purpose there are provided three contact electrodes **31, 32, 33,** the three of them integrated in the device, without the need to have linking wires like in most prior art devices.

The first electrode **31** is arranged on the internal wall **26** of the band and has a sensitive face oriented toward the skin of the arm. The first electrode **33** is also arranged on the internal wall **26** and has also a sensitive face oriented toward the skin of the arm.

Each of the electrodes is formed as a thin pad of a surface comprised between 5 $cm^2$ and 10 $cm^2$; for example, the shape of the thin pad is somewhat arched to follow the standard curvature of the skin of the arm.

In a particular option, first and third contact electrodes **31, 33** are disposed at distance from each other. Alternatively, first and third contact electrodes **31, 33** can be arranged differently, for example one above the other or one aside the other.

Whenever the armband is pressurized, first and third contact electrodes **31, 33** are firmly pressed against the skin of the arm, thereby ensuring a fairly good contact with a small electrical contact resistance. It should be noted that no gel is required at the contact electrode contrary to conventional habits. Contact electrodes are to be placed against the bare skin; however, thanks to the pressure, it is not excluded to have a light underwear cloth between the skin and the electrodes.

The contact electrodes can be made of stainless steel, silver, or other coated materials (coated with silver, chromium, gold, titanium or platinum), not excluding materials coated by physical vapor deposition technique (known as PVD techniques).

It is to be noted that two electrodes might be sufficient, therefore the third electrode **33** is considered optional.

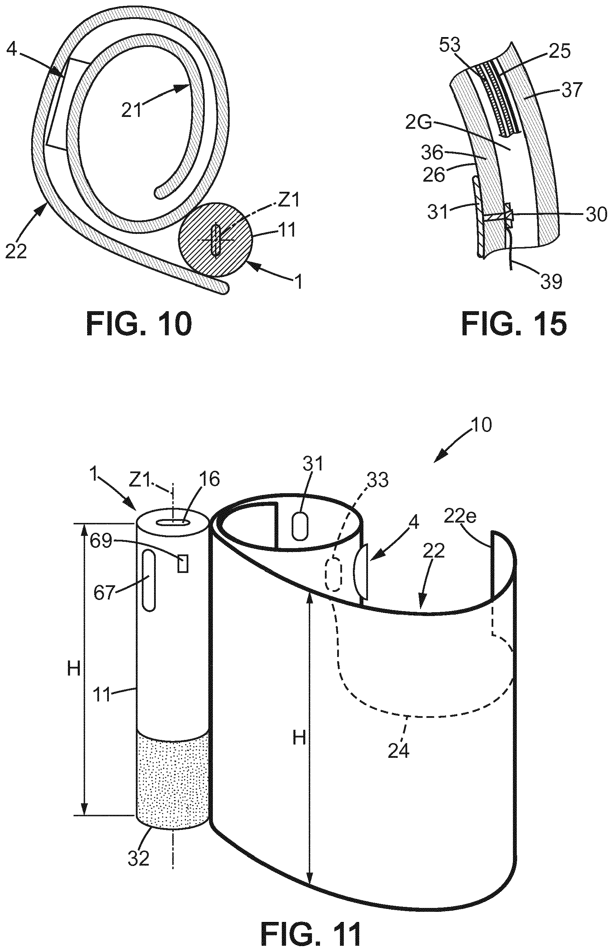

Regarding the second electrode **32,** it is arranged around the external surface of the control unit assembly as best seen at figure 11. A conductive material forms a coating of at least a part of the control unit housing. A metallic coating material (silver, titanium, chromium), are deposited by physical vapor deposition technique (known as PVD techniques).

The second electrode covers the lower third of the cylinder, for example all around the accessible circumference by the fingers of the user (see Fig 1). Therefore, it is easy for the user to grab/seize the second electrode with a good electrical contact.

The above mentioned control unit assembly **1** has, in the shown example, an overall cylindrical shape with an axis denoted **Z1** (cf Figures 2,4,11). The control unit assembly **1** is fixed to the arm band **2**. For example, it is fixed to the first portion **21** as explained below.

As seen on Figures, the general arrangement is as follows: the control unit assembly **1** extends from the external wall 27 of the band with regard to the main axis **Z** along a direction denoted **X**. In use configuration, when the chest of the user is nearly vertical, **X** is substantially horizontal and in a front-rear direction.

In the illustrated case of a cylinder, the diameter of the control unit assembly **1,** denoted **D1,** is less than 40 mm, for example about 35 mm or even about 30 mm.

The acoustic sensor **4** has a center **44,** which is referred to to define the transversal axis **Y,** extends from the external wall 27 of the band with regard to the main axis **Z** and passing through the center **44** of the acoustic head. In use configuration, when the chest of the user is nearly vertical, **Y** is substantially horizontal and in a left-right direction.

Angular distance between axis X and Y is denoted by angle $\alpha$, In use configuration, the angle $\alpha$ is comprised between 90° and 140°, for example between 110° and 130°. As shown at Figure 1, in use configuration, the right hand can conveniently seized the control unit assembly **1** and the acoustic sensor is naturally placed against the chest of the user **U.**

Regarding the acoustic sensor **4,** according to one preferred option, it is formed as a piezoelectric transducer, which can provide a very thin configuration; this piezoelectric transducer requires very little space projecting from the external wall **27** of the arm band; this piezoelectric transducer requires no electronic supply, no local electronic adaptation circuit.

Since the user naturally squeezes the acoustic sensor against the chest, the acoustic sensor can properly work through a thin cloth like a T-shirt, a shirt, even two layers of such cloth.

According to an alternative option, the acoustic sensor can be formed as a microphone.

In Figure 2, a generic view of the armband is represented; this type of band can be a ring adaptable in diameter/circumference. This kind of band can be inserted from the hand without opening the ring, and slid up to the shown position on the arm. There may be provided restriction means to decrease the play and lock the current position, before pneumatic inflation.

An inflatable bladder **53** is provided. Such a compliant inflatable bladder is known per se in blood pressure sensing apparatuses, and therefore not described in details here. At rest, the bladder is arranged within the thickness of the band, as an internal layer.

There may be provided an armature **25,** otherwise called *cuff holder*, for structural support of at least a part of the band. The armature can be an arcuate plastic part, made from a plastic material with good or high mechanical properties (polypropylene, ABS, PVC, or the like), having a part-of-a-cylinder shape, or generally an arcuate shape.

According to one particular aspect, both the bladder **53** and the armature **25** extend circumferentially along the major part of the active portion of the armband **2;** such that in use, the bladder is surrounding practically all the circumference of the arm of the user. Therefore, a homogeneous pressure is applied all around the arm which is beneficial for the accuracy of the measurement and the quality of the contact of the ECG electrodes **31,33.**

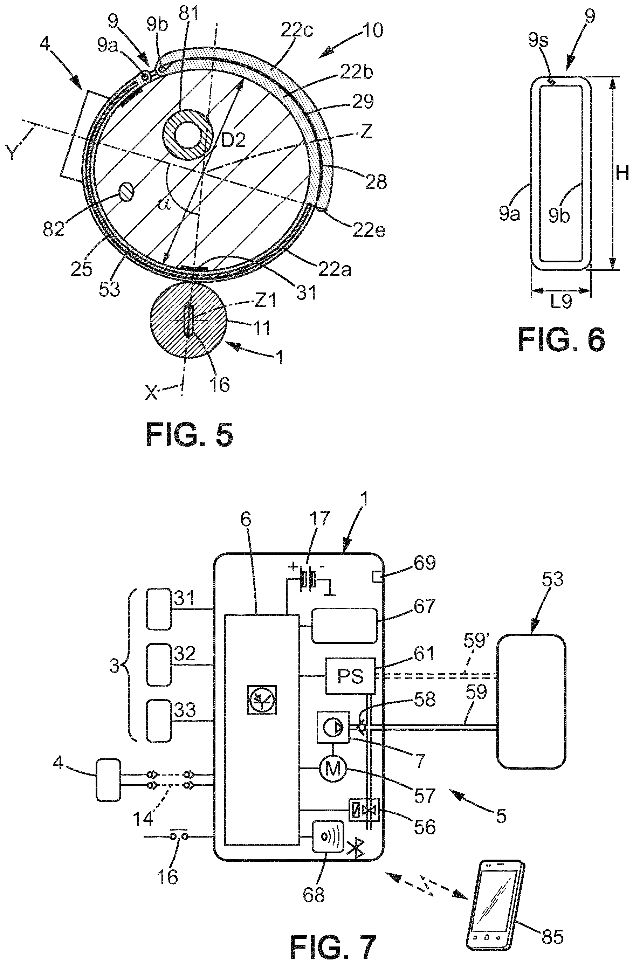

As visible on Figure 7, the control unit assembly **1** comprises a battery **17,** an electronic controller **6** and a pneumatic unit **5.** We note that no external wired connection is needed.

The pneumatic unit **5** comprises at least a pump **7** driven by an electric motor **57,** a release valve **56** also called "bleeder valve" **56,** and a pressure sensor **61.**

The pneumatic unit **5** may optionally comprise a check valve **58.** The release valve 56 may be an On/off valve or a progressive valve.

The control unit assembly **1** comprises an On/Off switch **16;** the user may start a measurement, after having installed the armband, by actuating the switch **16,** pressing or touching according to various possible types of switches.

Further the control unit assembly **1** may further comprise a wireless interface **68** such as for example a wireless coupler (WiFi, Bluetooth™, BLE or the like), and a display **67** already mentioned. The display **67** can be a LED display and or a dot matrix display; on this display, blood pressure results can be displayed directly without use of the smartphone application.

There is provided a pneumatic hose **59** to fluidly connect the output of the pump to the bladder. It can be a one-way pneumatic connection or a two-ways pneumatic connection (59,59'). According to one variant, there is provided a specific sense line **59'** decoupled from the pressurization line 59.

The pressure sensor 61 is in fluid connection with the inflatable bladder 53 through the specific line 59', or through the common single 59 where applicable.

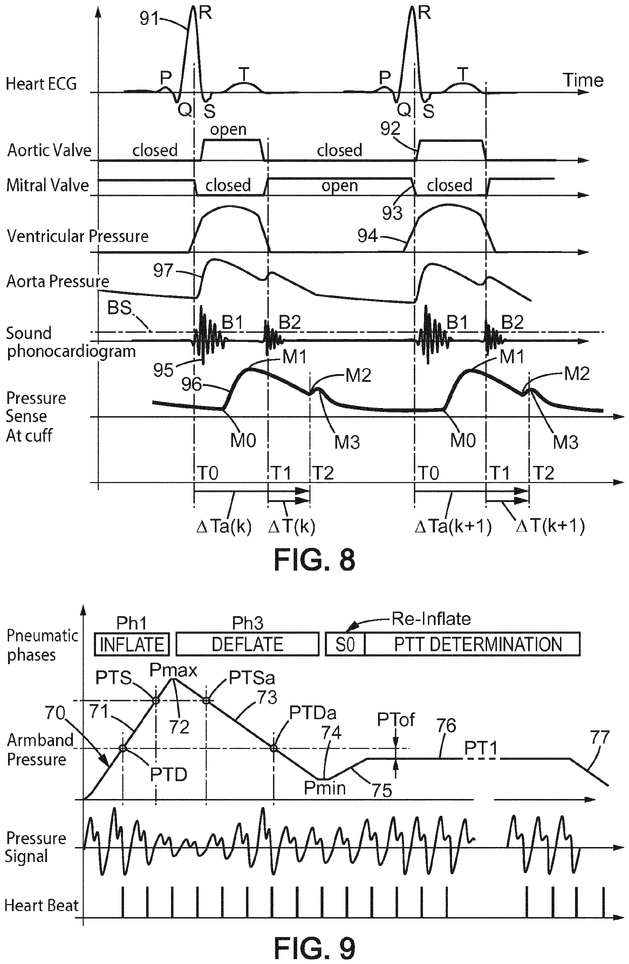

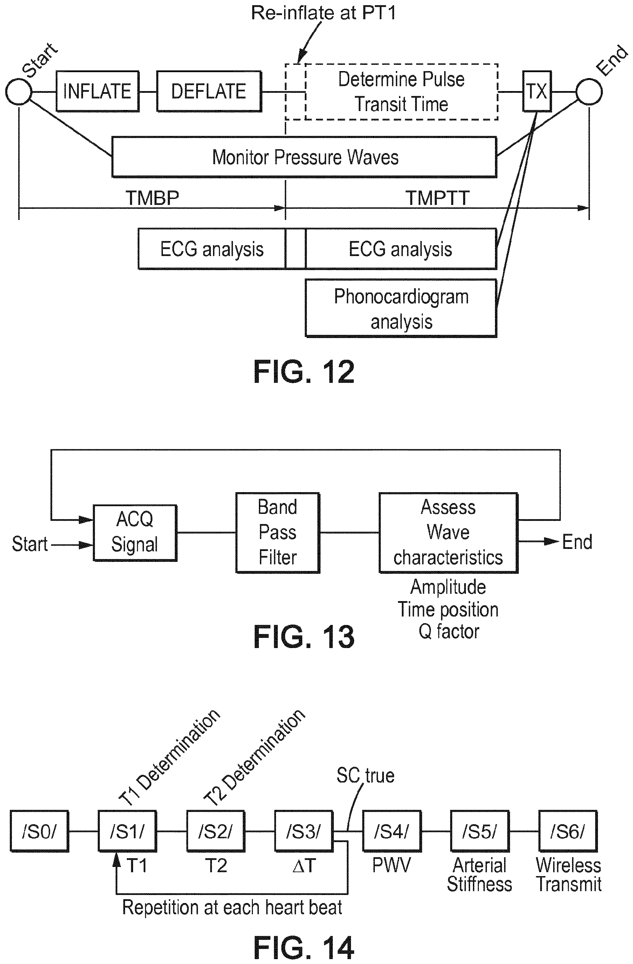

A first overview of the functionality of the device is given here, whereas it would be described later in more detail with the help of Figures 9 and 12.

A blood pressure measuring cycle is carried out first, and optionally, thereafter a pulse transit time PTT measuring cycle is carried out. In the same timeframe or separately, individual ECG signal analysis and/or phonocardiogram signal analysis can also be performed.

For the blood pressure measuring cycle, the electronic controller 6 is configured to first inflate the inflatable bladder 53 until the blood flow is greatly reduced by the pressure exerted on the arm. During inflation, the analysis of the evolution of pressure signals allows to infer the systolic pressure and the diastolic pressure. The controller is configured to then progressively deflate the bladder **53.** The progressive reinstatement of the blood pressure waves is also analyzed by the electronic controller **6** to infer the systolic pressure and the diastolic pressure, in confirmation or replacement of values deduced during the inflation phase.

Regarding the pulse transit time PTT, the electronic controller **6** determines a first characteristic instant and a second characteristic instant, and the resulting time difference is used to calculate a pulse wave velocity to finally issue an index representative of the arterial stiffness to the user.

Now are described in detail embodiments and variants of the device structure.

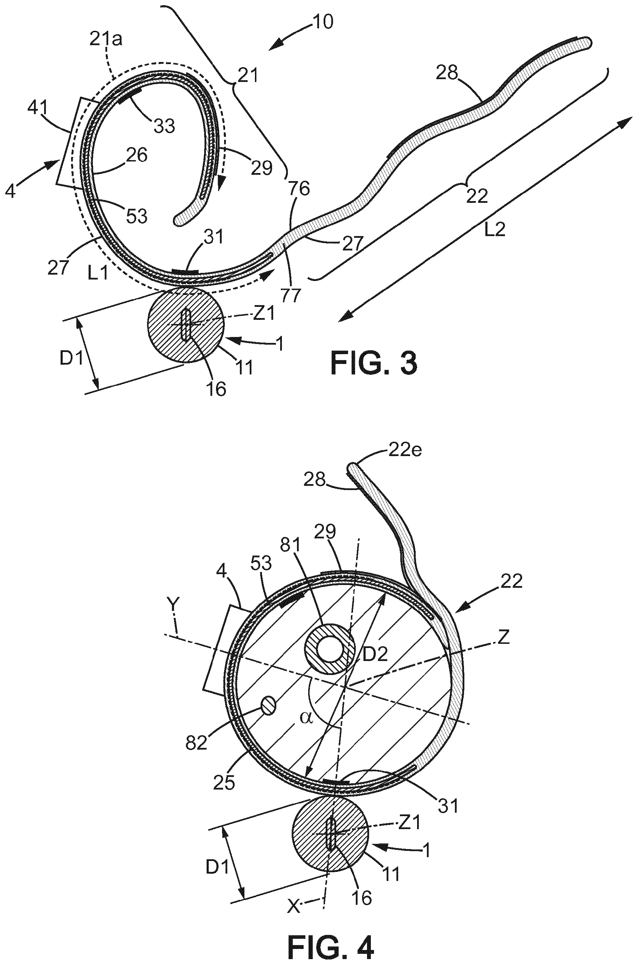

According to the first embodiment, illustrated at Figures 3, 4, 10 and 11, the band **2** comprises a first portion **21** and a second portion **22**. The first portion **21** can be considered as the main portion since the control unit assembly **1** and the acoustic sensor **4** are affixed to this first portion **21,** and furthermore this first portion houses the inflatable bladder **53,** and optionally, the structural elastic armature **25** already mentioned.

The first portion **21** has a developed length denoted **L1**. The second portion **22** has a developed length denoted **L2**. The band has a height **H**. Likewise, the armband **2** is made from a generally rectangular shape with a width corresponding to dimension **H** and a length corresponding to added dimensions **L1** + **L2.**

For installing the arm band **2** around the arm prior to inflating, the user starts for example from a stowed configuration depicted in figure 10, the user unrolls the second portion **22** (see Fig 3) to make it possible to install the first portion around his/her left arm BG. Thanks to the elasticity of the first portion, the first portion **21** can be opened so as to facilitate the insertion of the arm into the internal space encompassed by the first portion. The user has to move away a little bit his/her arm from the chest to do that.

It should be noted that this configuration allows installing the band/cuff without inserting it along the forearm from the hand side.

Further operation involves the closing of the band 2 around the arm, and securing this configuration prior to inflation.

A hook pad **29** is arranged at the external wall of the first portion **21** and a corresponding loop pad **28** is arranged at the internal wall of the second portion **22.** Of course the reverse configuration loop/hook is also possible.

Hook pad **29** and loop pad **28** may have a general rectangular shape. Regarding the dimensions, the hook pad **29** can extend along all the height **H** over a length of 5cm to 10cm; the loop pad **28** is longer, it also can extend along all the height **H** but over a length of 10cm to 20cm.

After the user has placed the first portion **21** around the arm on the side of the chest as described before, he/she pulls the second portion **22** toward the rear direction and then sticks the second portion 22 onto the first portion 21 by securing the loop pad **28** on the hook pad **29.** The remaining unused end 22e of the second portion is left free.

Alternatively, according to a variant illustrated at figure 11, where a special cutout 24 is provided, the remaining unused end **22e** of the second portion can be folded around back into the front direction.

Fig 4 shows the device ready for inflation, the user presses the device onto his/her chest to guarantee acoustic adaptation, namely proper collection of acoustic waves.

It should be noted that the length of the second portion together with the length of the hook pad and the length of the loop pad allows the device to be adjusted to a large variety of arm circumference and diameter **D2.**

At Figures 5 and 6, a second embodiment of the armband is represented, which also allows to instal the band/cuff without inserting it from the hand. Again, the band **2** comprises a first portion **21** and a second portion **22.**

In this embodiment, the device also comprises a buckle **9,** also named 'return buckle' as seen on Fig 6. The buckle can be manufactured as a bent metallic wire, shaped as a rectangular loop. The buckle can also be made from plastic material with good or high mechanical properties. Two long sides **9a, 9b** are connected at their respective ends by two small sides, thereby forming a rectangle with a length/height **H** and a width denoted **L9.**

The buckle is fastened to one circumferential end **21a** of the first portion 21, at complete opposite from the other end **22e** of the band 2.

For the assembly of the buckle on to the end **21a,** it can be provide a seam in the band end, done after insertion of the loop buckle. Alternatively, the loop formed by the buckle may have an openable slit **9s,** with self locking retaining means (hook and the like).

For installing the arm band **2** around the arm prior to inflating, the user places the first portion **21** around the arm on the side of the chest and passes the second portion **22** into the buckle **9** toward the rear and then pulls the end of the second portion **22** toward the front direction, until the arm wraps without substantial play the arm. Then the user sticks the returning portion **22c** against the base **22b** of the second portion to attach the attachment means, which results in the configuration shown at figure 5.

According to an aspect which is common to above embodiments, at least in part of the band, as shown at Figure 15, the band **2** comprises an internal layer **36** and an external layer **37** with an interval **2G** available between the two layers. In the gap **2G,** there may be provided the bladder **53,** the armature **25,** rivets **30** for fastening the contact electrode 31 and a connection wire **39.** Other wires may be present, for connecting the acoustic sensor to the electronic controller **6,** and the third electrode.

There may be a single layer portion (i.e. without gap or interval), notably in the distal end of the second portion 22.

The band **2** is for example made of strong fabric, woven or non-woven or synthetic material.

Generally speaking, the band **2** comprises attachment means, for fixing the size of the armband prior to inflating. The attaching means may comprise one or more couple(s) of loop and hook pads.

Therefore, a 'continuous' unquantized adjustment of encompassed circumference is made available, for any size of arm.

It should be noted that in the present specification, arm circumference **CIRC** or diameter D2 are indifferently used, since we know that **CIRC** = $\pi \times$ **D2.**

According to other possible solutions, there may be provided attaching means including a releasable ratchet system, or a teeth system.

Regarding dimensions, the following preferences can be noted.

For a baseline device intended to encompass arms having a perimeter/circumference comprised between 20cm and 42 cm, which represents most of the users :

> **L1** is for example comprised between 20 cm and 32 cm.
> **L2** is for example comprised between 15 cm and 25 cm.
> **H** is for example comprised between 12 cm and 16 cm.

For an XXL device (special large dimension variant) intended to encompass arms having a perimeter comprised between 40cm and 62 cm :

> **L1** is for example comprised between 40 cm and 45 cm.
> **L2** is for example comprised between 20 cm and 25 cm.
> **H** is for example comprised between 14 cm and 18 cm.

This XXL variant can have a frusto-conical configuration, smaller diameter at the elbow oriented end.

When the device is unused, as illustrated at figure 10, it can be rolled up, whereby its size is less than 10cm x 10cm x H.

In the variant illustrated at figure 11, the second portion 22 of the armband can exhibit a smaller height for its most distal area; this option is delineated by the dotted line **24;** in this case, when the free end **22e** of the second portion is wrapped around the first portion at the location of the acoustic sensor, the acoustic sensor 4 is still uncovered by the second portion. Therefore, even though the user installs the device by wrapping it all around, this won't prevent the device from working regarding the acoustic acquisition against the user's chest.

The device works as follows, as illustrated at figures 8, 9, 12, 13 and 14.

During a measurement, the patient's heart generates electrical impulses that pass through the body at high speed. Also simultaneously the patient's heart generates acoustic waves that pass through the body with a certain sonic speed.

These impulses/waves accompany each heartbeat, and the heartbeat generates a pressure wave in artery network that propagates through the patient's vasculature at a significantly slower speed. The blood path **P** of interest has a certain length, let's say 30 cm to 40 cm according to the physical characteristic of the individual under measurement. As will be seen in more detail later, this length depends at first order on user's height denoted **UH.**

Immediately after the heartbeat ventricular contraction, the pressure wave leaves the heart and aorta, passes through the subclavian artery, to the brachial artery along the path **P.**

The ECG electrodes measure electrical signals which pass to an amplifier/filter circuit within the control unit assembly. For example, a filtering circuit is provided before the signal is digitized and entered into the microcontroller.

Within the controller, the signals are processed with an analog-to-digital converter to form the ECG digitized waveform and then recorded together with the time of occurrence, namely instant **T0.** ECG waveform is named "QRS waveform" or "QRS complex" as sample shown at Figure 8.

The acoustic waves are also band-pass filtered and amplified, for example after upfront digitalization. A bandpass filter with cutting frequencies [0,5 Hz - 1kHz] is applied, either in the analog font end before digitization or applied to the digitized acoustic signal.

"QRS waveform" **91** is the top curve shown on timechart at Figure 8. This waveform is known per se thus not described in details here. Instant **T0** corresponds in the illustrative embodiment to R apex, but another marker can be taken alternately.

Aortic valve open/close state is also shown just beneath, signal denoted **92.**

Mitral valve open/close state is also shown just beneath, signal denoted **93.**

Just before aortic valve opening, the mitral valve closes; this produces a particular sound which is reflected in the first significant sound named **B1** as shown on curve **95.**

Further, after closing mitral valve and opening aortic valve, the ventricular volume decreases as blood is ejected to the aorta. At the same time ventricular pressure **94** exhibits a rounded apex. Aortic pressure curve is shown and denoted **97.**

Sound phonocardiogram corresponds to curve denoted **95** electrically reflects waves received at the acoustic sensor **4.**

This curve **95** exhibits two characteristics sounds; the first sound denoted **B1** corresponds to the closing of the mitral valve, the second sound denoted **B2** corresponds to the closing of the aortic valve.

A "*significant sound*", in the sense of the present disclosure, is defined whenever a instantaneous power of the acoustic signals exceeds a predetermined threshold (BS), cf Fig 8.

It should be noted that sounds **B1** and **B2** exceeds **BS** threshold.

Pressure wave at pressure sensor **61** in fluid communication with bladder **53** is shown at curve **96.**

This curve **96** exhibits three characteristics apexes. The first apex denoted **M1** is a maximum apex; the second apex denoted **M2** is a minimum local apex; the third apex denoted **M3** is a maximum local apex.

Besides **M0** is the minimum value, just before the rise which is a consequence/response of the arrival of the pressure pulse at the arm.

The second apex denoted **M2** is a marker corresponding to arrival of the effect of the closure of aortic valve at the brachial artery within the arm band.

There may be defined a reference point in the arm, so that the ideal position for the device can be notified to the user, for example distance from the elbow internal fold, or another criterion.

Generally speaking, for the purpose of PTT, **T1** is defined as the instant of the maximum instantaneous signal power of the second sound **B2** reflecting when aortic valve closes.

Generally speaking, **T2** is defined as the instant when second apex denoted **M2** occurs.

Blood Pressure procedure

This procedure is known in the art, and thus it is not described in details here. Basically, it comprises the following phases:

/Ph1/- start inflating the bladder, inflation phase is denoted **71** at Fig 9, '*INFLATE*' at Fig 12,
/PhD/ - determining Diastolic Blood pressure (PTD) during inflating phase
/PhS/ - determining Systolic Blood pressure (PTS), during inflating phase
/Ph2/- stop inflating the bladder **72** (when hardly no more pressure wave is identified),
/Ph3/- start deflating the bladder, deflation phase is denoted **73** '*DEFLATE*' at Fig 12,
/PhS/ - determining Systolic Blood pressure (PTSa), during deflating phase
/PhD/ - determining Diastolic Blood pressure (PTDa) during deflating phase

More precisely, the shape of the pressure waves are analyzed by the electronic controller. During inflation **71,** as shown at Fig 9, the shape of the pressure waves evolves, and a predefined criteria on the waveform makes the decision to record a first diastolic blood pressure **PTD,** and another set of predefined criteria on the waveform makes the decision to record a systolic blood pressure **PTS.**

In a similar manner, during deflation phase, the shape of the pressure waves are analyzed by the electronic controller. During deflation **73** the shape of the pressure waves evolves, and a predefined criteria on the waveform makes the decision to record another systolic blood pressure **PTSa,** and another set of predefined criteria on the waveform makes the decision to record another diastolic blood pressure **PTDa.**

It should be noted that pressure curved is shown after rectifying at figure 8, whereas it is shown before rectifying at figure 9.

The second systolic blood pressure noted **PTSa** can be regarded as a confirmation of the first systolic blood pressure noted **PTS.** According to one example, an outputted systolic blood pressure can be an average of the value PTS and PTSa.

Similarly, the second diastolic blood pressure noted **PTDa** can be regarded as a confirmation of the first diastolic blood pressure noted **PTD.** According to one example, an outputted diastolic blood pressure can be an average of the value PTD and PTDa.

PTT Procedure

This procedure is adapted to determine as accurately as possible the pulse transit time PTT.

It comprises the following phases:

/S0/ - inflate **75** the bladder at a predetermined pressure denoted **PT1,** the inflation bringing the pressure from the lowest value **74** to this predefined level **PT1,** detailed below
/S1/- determining the above-mentioned aortic valve closing instant **T1**(k) from acoustic signals,
/S2/- determining subsequently, from pressure signals, a characteristic point (M2) of the pressure signal curve occurring at instant **T2**(k),
/S3/- calculate a time difference, defined as $\Delta T(k) = T2(k) - T1(k)$

The predetermined pressure **PT1** can be defined as a function of **PTD,** for example with a value below the diastolic pressure **PTD;** this value may be defined by a predefined offset **PTof;** in other words, PT1 can be such **PT1 = PTD - PTof,** with **PTof** for example equal to 10 mmHg (10 Torr).

According to one option, said characteristic point (**M2**) is the above-mentioned local minimum apex, after first apex **M1**.

Further, steps **S1** to **S3** are repeated until a stop criterion **SC** is met. This stop criterion **SC** can be defined according to different possibilities. One consists in predefined duration. Another one consists in counting the number **N** of heartbeat cycle ; for example **N** is chosen between 4 and 20, for example between 6 and 12.

Overall duration for BP procedure is denoted **TMBP** and duration for pulse transit time procedure is denoted **TMPTT**. For example **TMBP** is less than 10 seconds. For example **TMPTT** is less than 12 seconds. According to a user configuration, selectively chosen by user through the smartphone or locally by a double press on the switch **16,** the user can choose to carry out only the blood pressure **or BP** procedure plus **PTT** procedure.

During PTT procedure, pump **7** is not energized and a bleeder valve comprised in the pneumatic unit is not energized. Therefore, no intrinsic parasitic noise disturbs the phonocardiogram analysis and therefore the PTT procedure and related calculation is not disturbed by endogenous noise.

The electronic controller **6** computes therefrom a time difference, defined as

> $\Delta$**T(k) = T2(k) - T1(k)** for the heartbeat arbitrarily numbered **k.**
> **PWV** is the wave velocity along path P.
> **PWV** is defined as **PWV = length(P) /** $\Delta$**T(k)**

In practice, we prefer to rely on a successive series of **N** measurements; in this condition, the method may comprise the following:

> /S41/ - calculate an average value $\Delta$**Tav** of $\Delta$**T(k),** for **k=j to j+N**
> /S42/- calculate a Pulse Wave Velocity (PWV) defined as **PWV(k) = length(P) /** $\Delta$**Tav,**

The number **N** of effective PTT measurement can be chosen between 4 and 20.

The average value can be computed as follows

$$\Delta \mathbf{Tav} = \frac{1}{N} \sum_{k=j+1}^{k=j+N} \Delta \mathbf{T(k)}$$

The **height** (UH) of the user is taken into account at step S42, namely **length(P) = F1 (UH)**

Also any combination of **age** (UA), **gender** (UG), and **weight** (UW), of the user may additionally be taken into account at step /S42/, namely **length(P) = F2 (UH,UA,UG,UW).**

Arterial stiffness **AS** can be defined as a function of **PWV**. AS can be expressed as a rating between 1 and 10 ; it can also be expressed as an equivalent age of the person. Arterial stiffness **AS** is referred to as step /**S5/**.

Fig 14 shows a summary of the above mentioned steps /S0/ to /S5/, with a repetition until stop criterion (SC) becomes true (in other words criterion is met).

An optional step referred to as step /S6/ consists in transmitting the resulting data and parameters to the smartphone 85 over the wireless link. Please note that alternatively this transmission can be continuous all along the process, ECG signal and/or phonocardiogram can be displayed in real time on the smartphone application. Transmission of resulting data and parameters can also be done by packets, for example one packet after blood pressure values are obtained (BP procedure) and another packet after PTT Procedure.

A plurality of subsequent evaluations of Pulse Wave Velocity are recorded for a particular user to form a history curve, and a deviation in said curve is notified to said particular user.

It should be noted that the disclosed device has complete power autonomy; According to a particular option, the device is formed as an integral unit, there is no external wire, not external hose, therefore style and practicality are enhanced.

Summary of Cardiovascular parameters/functions that can be monitored by the device :

As already mentioned, Diastolic blood pressure **PTD,** and systolic blood pressure **PTS** can be obtained from the blood pressure procedure, as a motion of blood pressure cuff.

Also, as described before, PTT procedure allows to determine the arterial stiffness.

Also, heart rate **HR** can be inferred from ECG signals 91, from phonocardiogram signals 95, or from oscillometric signals 96, by measuring the average time separation between two heartbeats and deducing therefrom the number of heartbeat and minute.

HR variability is also calculated, over at least three subsequent heartbeats.

Also, the device can perform ECG signal analysis, for detection of arrhythmia, as known per se. Some anomalies in ECG signal 91 can be identified and associated to a type of arrhythmia, such as arterial fibrillation.

Also, the device can perform a phonocardiogram analysis for detection of heart anomalies responsible for so-called heart murmurs. In particular, some anomalies in phonocardiogram signal 95 can be identified and associated to a valvulopathy, concerning any of the mitral, tricuspide, aortic, or pulmonary valves.

ECG analysis and phonocardiogram analysis can be carried out simultaneously with the PTT procedure, or separately, namely before or after PTT procedure.

As illustrated at figure 12, ECG analysis can be performed all along and at any time. According to one option, though ECG analysis is suspended whenever the electric motor is energized to run the pump (this produce electromagnetic interference detriment as to ECG signals).

Regarding the phonocardiogram analysis, it is for example carried out when no intrinsic source of noise is present, for example during the constant pressure sequence for PTT determination.

As shown at figure 13, pressure signals are sensed by pressure sensor **61** and made available at the microcontroller **6**. According to one option the microcontroller 6 digitizes the signal and then applies a digital bandpass filter. For example a band range of [0,3Hz - 3kHz] or [0,5Hz - 1kHz] can be used.

The microcontroller **6** identifies wave pulses and determines values characteristics of said pulses, i.e. amplitude, time position, apexes, derivatives, Q-factor,...

System, application and remote patient monitoring

The user can follow his/her own metrics on a smartphone application.

The device can be used by more than one user, selection of relevant user can be done through the local display 67 or through the smartphone application. When actuated, directly on the device, the activation switch 16 can be used to scroll across several users names.

There is provided a Micro USB connection **69** for battery recharge and for up/downloading of data.

The smartphone application can display a history of measurement reports, which can include BP, PTT, ECG signals, phonocardiograms.

It should be noted that phonocardiogram can be replayed via the smartphone application and the loudspeaker/headphones coupled to the smartphone.

Smartphone application can issue time reminders for the user, so the user can measure cardiovascular parameters regularly with the integrated device **10.**

The integrated device **10** is configured to send an alert to the physician whenever some particular thresholds are exceeded or when particular events are detected, such as an episode of arrhythmia. Such thresholds regarding blood pressure can be defined either by the user him/herself or by the physician/doctor.

The user can add the contextual note(s), such as medicine intake or a life circumstance (just after wakeup, just before going to bed), to one or more measurement reports.

Measurement reports can be sent remotely to a server that can be accessed the physician/doctor, so that the physician can analyze the patient's data from a distant location.

The system allows remote analysis of ECG signals; the user can receive in return the diagnostic from the physician.

Although the drawings and the text above mainly focus on an example with an armband placed around the left arm, it should be understood that the predefined position where pressure signals are captured could also be somewhere else on one limb of the user, for example at the forearm, at the wrist, somewhere else on the right arm of the user, including the wrist; it is not excluded to implement the proposed method on a lower limb of the user. Of course, parameters to be used are to be adapted in particular to the length of the arterial blood path P.

Also, it is important to note that the acoustic sensor can be placed somewhere else other than the left side chest of the user, the acoustic sensor may be provided with an extension cord to electrically couple the acoustic sensor to the control unit.

Additionally, there may be provided a control unit implemented differently, not necessarily adjacent to the armband.

According to another embodiment, instead of an inflatable bladder, the system may include pressure exerting means that are different from compressed air. For example, it can be two bladders filled with water with a ballasting system. As per another example, it can be a mechanically collar with controllable restraint. Although, solutions like a set of piezoelectric actuators, or a collar with temperature dependent memory alloys are not excluded.

According to still another embodiment, instead of a pressure sensor, at least one blood circulation parameter like the instantaneous local speed can be sensed by a Doppler effect sensor placed adjacent to a surface blood vessel.

**Claims**

1.  A method to collect cardiovascular data relating to an individual user (U), in a system comprising an acoustic sensor (4) configured to be coupled to the chest of the user, an arm band (2) having an inflatable bladder (53) configured to be placed at a predefined position around a limb of the user, for example at the left arm (BG), a pneumatic unit with at least a pump (7) configured to inflate and deflate the inflatable bladder, and a pressure sensor (61), an arterial blood path (P) being defined from the heart to the predefined position, the method comprising a set of steps named PTT procedure :

    /A1/- acquiring acoustic signals at the acoustic sensor,
    /A2/- acquiring of blood pressure signals at the pressure sensor, image of instantaneous blood pressure prevailing at the predefined position,
    /S1/- determining aortic valve closing instant T1(k) from acoustic signals, the aortic valve closing instant T1(k) being defined as a second significant sound (B2) of the heartbeat,
    /S2/- determining subsequently, from blood circulation signals, a characteristic point (M2) occurring at instant T2(k), the characteristic point (M2) of the pressure signal curve being defined as a succession of a maximum apex (M1) and a minimum apex (M2), said instant T2(k) being defined as the instant when said minimum apex occurs,
    /S3/- calculate a time difference, defined as

$\Delta T(k) = T2(k) - T1(k)$
/Sloop/- repeating, for each heartbeat, steps /S1/ to /S3/ until a stop criterion (SC) is met.

2. The method according to claim 1, wherein the method comprises a step of:

/S0/ - exert a predetermined pressure (PT1) below the diastolic pressure (PTD), on the limb, wherein, during PTT procedure, pump is not energized and a bleeder valve comprised in the pneumatic unit is not energized.

3. The method according to claim 1 or 2, wherein at step /S2/ the characteristic point (M2) is determined from a pressure signal curve.

4. The method according to claim 3, wherein the means for exerting pressure is a band (2) having an inflatable bladder (53) configured to be placed at a predefined position around a user's arm.

5. The method according to claim 4, wherein, the predefined position is at the left arm (BG) of the user, the band (2) is an arm band and is configured to be placed around the left arm of the user.

6. The method according to any of the claims 1 to 5, wherein the system comprises a set of contact electrodes (3) for electrocardiographic sensing, configured to be brought in contact with the skin of the user U, the method comprising the steps:

/A3/- acquiring ECG signals at the contact electrodes,
/S10/ - determining a characteristic QRS signal from ECG signals as a synchronization signal (T0) reflecting heartbeat.

7. The method according to any of claims 1 to 6, wherein at step /S1/, the aortic valve closing instant T1(k) is determined as follows:

/S11/- identifying a first significant sound (B1) of the heartbeat, reflecting mitral valve closing, just following QRS signal at T0,
/S12/- identifying a second significant sound (B2) of the heartbeat, reflecting aortic valve closing, and record said second significant sound (B2) as instant T1(k).

8. The method according to any of the claims 1 to 7, wherein the method comprises, before the PTT procedure, a preliminary set of steps named Blood Pressure procedure comprising the steps:

/Ph1/- start inflating the bladder,
/Ph2/- stop inflating the bladder (when no more pressure wave is identified),
/Ph3/- start deflating the bladder,
meanwhile are performed the following steps:

/PhS/ - determining Systolic Blood pressure (PTS), during inflating phase and/or deflating phase
/PhD/ - determining Diastolic Blood pressure (PTD), during inflating phase and/or deflating phase

9. The method according to claim 8, wherein prior to steps /S1/ to /S3/, the predetermined pressure PT1, providing pressurization for PTT procedure, is calculated with reference to the diastolic pressure (PTD) determined at step /PhD/.

10. The method according to claim 9, wherein the predetermined pressure (PT1) is defined as a function of PTD and a predefined offset (PTOf), for example such PT1 = PTD - PTof, with PTof for example equal to 10 mmHg (10 Torr).

11. The method according to any of the claims 1 to 10, further comprising:

/S41/ - calculate an average value $\Delta Tav$ of $\Delta T(k)$, for k=j to j+N
/S42/- calculate a Pulse Wave Velocity (PWV) defined as PWV(k) = length(P) / $\Delta Tav$,
/S5/- assess therefrom an arterial stiffness (AS) of the user.

12. The method according to claim 11, wherein the height (UH) of the user is taken into account at step /S42/, namely length(P) = F1 (UH).

13. The method according to claim 11, wherein subsequent evaluations of Pulse Wave Velocity are recorded for a particular user (U) to form a history curve, and a deviation in said curve is notified to said particular user.

14. A system comprising an acoustic sensor (4) configured to be coupled to the chest of the user, an arm band (2) having an inflatable bladder (53) configured to be placed at a predefined position around a limb of the user, for example at the left arm (BG), a pneumatic unit with at least a pump (7) configured to inflate and deflate the inflatable bladder, and a pressure sensor (61), an arterial blood path (P) being defined from the heart to the predefined position, wherein
the system is configured to carry out the method comprising a set of steps named PTT procedure:

/A1/- acquiring acoustic signals at the acoustic sensor,

/A2/- acquiring of blood pressure signals at the pressure sensor, image of instantaneous blood pressure prevailing at the predefined position,

/S1/- determining aortic valve closing instant T1(k) from acoustic signals, the aortic valve closing instant T1(k) being defined as a second significant sound (B2) of the heartbeat,

/S2/- determining subsequently, from blood circulation signals, a characteristic point (M2) occurring at instant T2(k), the characteristic point (M2) of the pressure signal curve being defined as a succession of a maximum apex (M1) and a minimum apex (M2), said instant T2(k) being defined as the instant when said minimum apex occurs,

/S3/- calculate a time difference, defined as $\Delta T(k) = T2(k) - T1(k)$ /Sloop/- repeating, for each heartbeat, steps /S1/ to /S3/ until a stop criterion (SC) is met.

**15.** A computer readable medium encoded with instructions that, when executed by a computer, cause performance of a method according to any of the claims 1 to 13.

**Patentansprüche**

**1.** Verfahren zum Sammeln von kardiovaskulären Daten eines einzelnen Benutzers (U) in einem System, das einen akustischen Sensor (4), der so konfiguriert ist, dass er mit der Brust des Benutzers gekoppelt werden kann, ein Armband (2) mit einer aufblasbaren Blase (53), die so konfiguriert ist, dass sie an einer vordefinierten Position um ein Glied des Benutzers platziert werden kann, zum Beispiel am linken Arm (BG), eine pneumatische Einheit mit mindestens einer Pumpe (7), die so konfiguriert ist, dass sie die aufblasbare Blase aufpumpt und entleert, und einen Drucksensor (61), wobei ein arterieller Blutpfad (P) vom Herzen zu der vordefinierten Position definiert ist, wobei das Verfahren eine Reihe von Schritten umfasst, die PTT-Prozedur genannt werden:

/A1/- Erfassung der akustischen Signale am Akustiksensor,

/A2/- Erfassung der Blutdrucksignale am Drucksensor, Bild des momentanen Blutdrucks an der vordefinierten Position,

/S1/- Bestimmung des Schließzeitpunkts der Aortenklappe T1(k) aus akustischen Signalen, wobei der Schließzeitpunkt der Aortenklappe T1(k) als ein zweiter signifikanter Ton (B2) des Herzschlags definiert ist,

/S2/-, die anschließend aus den Blutkreislaufsignalen einen charakteristischen Punkt (M2) bestimmt, der zum Zeitpunkt T2(k) auftritt, wobei der charakteristische Punkt (M2) der Drucksignalkurve als eine Folge eines maximalen Scheitelpunkts (M1) und eines minimalen Scheitelpunkts (M2) definiert ist, wobei der Zeitpunkt T2(k) als der Zeitpunkt definiert ist, an dem der minimale Scheitelpunkt auftritt,

/S3/- eine Zeitdifferenz berechnen, definiert als $\Delta T(k) = T2(k) - T1(k)$

/Schleife/-, die für jeden Herzschlag die Schritte /S1/ bis /S3/ wiederholt, bis ein Stoppkriterium (SC) erfüllt ist.

**2.** Verfahren nach Anspruch 1, wobei das Verfahren einen Schritt umfasst:

/S0/ - einen vorbestimmten Druck (PT1) unterhalb des diastolischen Drucks (PTD) auf die Extremität ausüben,

wobei während der PTT-Prozedur die Pumpe nicht mit Energie versorgt wird und ein in der Pneumatikeinheit enthaltenes Entlüftungsventil nicht mit Energie versorgt wird.

**3.** Verfahren nach Anspruch 1 oder 2, wobei in Schritt /S2/ der charakteristische Punkt (M2) aus einer Drucksignalkurve bestimmt wird.

**4.** Verfahren nach Anspruch 3, wobei das Mittel zum Ausüben von Druck ein Band (2) mit einer aufblasbaren Blase (53) ist, die so konfiguriert ist, dass sie in einer vordefinierten Position um den Arm eines Benutzers gelegt werden kann.

**5.** Verfahren nach Anspruch 4, wobei die vordefinierte Position am linken Arm (BG) des Benutzers *liegt*, das Band (2) ein Armband ist und so konfiguriert ist, dass es um den linken Arm des Benutzers gelegt wird.

**6.** Verfahren nach einem der Ansprüche 1 bis 5, wobei das System einen Satz von Kontaktelektroden (3) zur elektrokardiographischen Abtastung umfasst, die so konfiguriert sind, dass sie mit der Haut des Benutzers U in Kontakt gebracht werden, wobei das Verfahren die Schritte umfasst:

/A3/- Erfassung der EKG-Signale an den Kontaktelektroden,

/S10/ - Bestimmung eines charakteristischen QRS-Signals aus EKG-Signalen als Synchronisationssignal (T0), das den Herzschlag widerspiegelt.

**7.** Verfahren nach einem der Ansprüche 1 bis 6, wobei in Schritt /S1/ der Schließzeitpunkt der Aortenklappe T1(k) wie folgt bestimmt wird:

/S11/- zur Identifizierung eines ersten signifikanten Tons (B1) des Herzschlags, der das Schließen der Mitralklappe widerspiegelt, unmittelbar nach dem QRS-Signal bei T0,

/S12/- Identifizierung eines zweiten signifikanten Tons (B2) des Herzschlags, der das Schließen der Aortenklappe widerspiegelt, und Aufzeichnung des zweiten signifikanten Tons (B2) als Zeitpunkt T1(k).

8. Verfahren nach einem der Ansprüche 1 bis 7, wobei das Verfahren vor dem PTT-Verfahren einen vorbereitenden Satz von Schritten umfasst, der als Blutdruckverfahren bezeichnet wird und die Schritte umfasst:

/Ph1/- beginnen, die Blase aufzupumpen,
/Ph2/- Beenden Sie das Aufblasen der Blase (wenn keine Druckwelle mehr erkannt wird),
/Ph3/- die Entleerung der Blase beginnen,
In der Zwischenzeit werden die folgenden Schritte durchgeführt:

/PhS/ - Bestimmung des systolischen Blutdrucks (PTS), während der Aufblasphase und/oder der Entleerungsphase
/PhD/ - Bestimmung des diastolischen Blutdrucks (PTD), während der Aufblasphase und/oder der Entleerungsphase

9. Verfahren nach Anspruch 8, wobei vor den Schritten /S1/ bis /S3/ der vorbestimmte Druck PT1, der die Druckbeaufschlagung für das PTT-Verfahren bereitstellt, unter Bezugnahme auf den in Schritt /PhD/ bestimmten diastolischen Druck (PTD) berechnet wird.

10. Verfahren nach Anspruch 9, bei dem der vorbestimmte Druck (PT1) als Funktion von PTD und einem vordefinierten Offset (PTOf) definiert ist, z.B. PT1 = PTD - PTof, wobei PTof z.B. gleich 10 mmHg (10 Torr) ist.

11. Verfahren nach einem der Ansprüche 1 bis 10, ferner umfassend:

/S41/ - Berechnung eines Durchschnittswerts ΔTav von ΔT(k), für k=j bis j+N
/S42/- eine Pulswellengeschwindigkeit (PWV) berechnen, die definiert ist als PWV(k) = Länge(P) / ΔTav,
/S5/- daraus eine arterielle Steifigkeit (AS) des Benutzers zu ermitteln.

12. Verfahren nach Anspruch 11, wobei die Höhe (UH) des Benutzers im Schritt /S42/ berücksichtigt wird, nämlich Länge(P) = F1 (UH).

13. Verfahren nach Anspruch 11, wobei nachfolgende Auswertungen der Pulswellengeschwindigkeit für einen bestimmten Benutzer (U) aufgezeichnet werden, um eine Verlaufskurve zu bilden, und eine Abweichung in der Kurve dem bestimmten Benutzer mitgeteilt wird.

14. System mit einem akustischen Sensor (4), der so konfiguriert ist, dass er mit dem Brustkorb des Benutzers gekoppelt werden kann, einem Armband (2) mit einer aufblasbaren Blase (53), die so konfiguriert ist, dass sie an einer vordefinierten Position um eine Gliedmaße des Benutzers, zum Beispiel am linken Arm (BG), platziert werden kann, einer pneumatischen Einheit mit mindestens einer Pumpe (7), die so konfiguriert ist, dass sie die aufblasbare Blase aufbläst und entleert, und einem Drucksensor (61), wobei ein arterieller Blutpfad (P) vom Herzen zur vordefinierten Position definiert ist,

wobei das System so konfiguriert ist, dass es das Verfahren, das eine Reihe von Schritten mit der Bezeichnung PTT-Verfahren umfasst, ausführt:

/A1/- Erfassung der akustischen Signale am Akustiksensor,
/A2/- Erfassung der Blutdrucksignale am Drucksensor, Bild des momentanen Blutdrucks an der vordefinierten Position,
/S1/- Bestimmung des Schließzeitpunkts der Aortenklappe T1(k) aus akustischen Signalen, wobei der Schließzeitpunkt der Aortenklappe T1(k) als ein zweiter signifikanter Ton (B2) des Herzschlags definiert ist,
/S2/-, die anschließend aus den Blutkreislaufsignalen einen charakteristischen Punkt (M2) bestimmt, der zum Zeitpunkt T2(k) auftritt, wobei der charakteristische Punkt (M2) der Drucksignalkurve als eine Folge eines maximalen Scheitelpunkts (M1) und
eines minimalen Scheitelpunkts (M2) definiert ist, wobei der Zeitpunkt T2(k) als der Zeitpunkt definiert ist, an dem der minimale Scheitelpunkt auftritt,
/S3/- eine Zeitdifferenz berechnen, definiert als ΔT(k) = T2(k) - T1(k)
/Sloop/- Wiederholung der Schritte /S1/ bis /S3/ für jeden Herzschlag, bis ein Stoppkriterium (SC) erfüllt ist.

15. Computerlesbares Medium, das mit Anweisungen kodiert ist, die, wenn sie von einem Computer ausgeführt werden, die Durchführung eines Verfahrens nach einem der Ansprüche 1 bis 13 bewirken.

**Revendications**

1. Méthode de collecte de données cardiovasculaires relatives à un utilisateur individuel (U), dans un système comprenant un capteur acoustique (4) configuré pour être couplé à la poitrine de l'utilisateur, un brassard (2) comportant une vessie gonflable (53) configurée pour être placée à une position prédéfinie autour d'un membre de l'utilisateur, par exemple au niveau du bras gauche (BG), une unité pneumatique comportant au moins une pompe (7) configurée pour gonfler et dégonfler la vessie gonflable, et un capteur de pression (61), par exemple au niveau du bras gauche (BG), une unité pneumatique avec au moins une pompe (7) configurée pour gonfler et dégonfler la vessie gonflable, et un capteur de pression (61), un trajet sanguin artériel (P) étant défini depuis le coeur jusqu'à la position prédéfinie, la méthode comprenant un ensemble d'étapes nommées procédure PTT :

/A1/- acquérir des signaux acoustiques au niveau du capteur acoustique,

/A2/- acquisition des signaux de pression sanguine au niveau du capteur de pression, image de la pression sanguine instantanée prévalant à la position prédéfinie,

/S1/- déterminer l'instant de fermeture de la valve aortique T1(k) à partir des signaux acoustiques, l'instant de fermeture de la valve aortique T1(k) étant défini comme un deuxième son significatif (B2) du battement cardiaque,

/S2/- déterminer ensuite, à partir des signaux de circulation sanguine, un point caractéristique (M2) survenant à l'instant T2(k), le point caractéristique (M2) de la courbe du signal de pression étant défini comme une succession d'un sommet maximal (M1) et d'un sommet minimal (M2), ledit instant T2(k) étant défini comme l'instant où se produit ledit sommet minimal,

/S3/- calculer une différence de temps, définie comme $\Delta T(k) = T2(k) - T1(k)$

/Sloop/- en répétant, pour chaque battement de coeur, les étapes /S1/ à /S3/ jusqu'à ce qu'un critère d'arrêt (SC) soit atteint.

2. Méthode selon la revendication 1, dans laquelle la méthode comprend une étape consistant à :

/S0/ - exercer sur le membre une pression prédéterminée (PT1) inférieure à la pression diastolique (PTD),

dans lequel, pendant la procédure PTT, la pompe n'est pas alimentée et une vanne de purge comprise dans l'unité pneumatique n'est pas alimentée.

3. Méthode selon la revendication 1 ou 2, dans laquelle à l'étape /S2/ le point caractéristique (M2) est déterminé à partir d'une courbe de signal de pression.

4. Méthode selon la revendication 3, dans laquelle le moyen d'exercer une pression est une bande (2) comportant une vessie gonflable (53) configurée pour être placée à une position prédéfinie autour du bras de l'utilisateur.

5. Méthode selon la revendication 4, dans lequel la position prédéfinie se *situe* au niveau du bras gauche (BG) de l'utilisateur, le bracelet (2) est un brassard et est configuré pour être placé autour du bras gauche de l'utilisateur.

6. Méthode selon l'une des revendications 1 à 5, dans lequel le système comprend un ensemble d'électrodes de contact (3) pour la détection électrocardiographique, configurées pour être mises en contact avec la peau de l'utilisateur U, le procédé comprenant les étapes :

/A3/- l'acquisition de signaux ECG au niveau des électrodes de contact,

/S10/ - détermination d'un signal QRS caractéristique à partir des signaux ECG en tant que signal de synchronisation (T0) reflétant le rythme cardiaque.

7. Méthode selon l'une des revendications 1 à 6, dans laquelle à l'étape /S1/, l'instant de fermeture de la valve aortique T1(k) est déterminé comme suit :

/S11/- identifiant un premier son significatif (B1) du rythme cardiaque, reflétant la fermeture de la valve mitrale, juste après le signal QRS à T0,

/S12/- identifier un second son significatif (B2) du rythme cardiaque, reflétant la fermeture de la valve aortique, et enregistrer ce second son significatif (B2) en tant qu'instant T1(k).

8. Méthode selon l'une des revendications 1 à 7, dans laquelle la méthode comprend, avant la procédure PTT, une série préliminaire d'étapes appelée procédure de pression artérielle comprenant les étapes suivantes :

/Ph1/- commencent à gonfler la vessie,

/Ph2/- arrêter de gonfler la vessie (lorsque plus aucune onde de pression n'est identifiée),

/Ph3/- commencer à dégonfler la vessie,

entre-temps, les étapes suivantes sont réalisées :

/PhS/ - détermination de la pression artérielle systolique (PTS), pendant la phase de gonflage et/ou la phase de dégonflage

/PhD/ - détermination de la pression artérielle diastolique (PTD), pendant la phase de gonflage et/ou la phase de dégonflage

9. Méthode selon la revendication 8, dans laquelle avant les étapes /S1/ à /S3/, la pression prédéterminée PT1, fournissant la pressurisation pour la procédure PTT, est calculée par rapport à la pression diastolique (PTD) déterminée à l'étape /PhD/.

10. Méthode selon la revendication 9, dans laquelle la pression prédéterminée (PT1) est définie en fonction de PTD et d'un décalage prédéfini (PTOf), par exemple PT1 = PTD - PTof, PTof étant par exemple égal à 10 mmHg (10 Torr).

11. La méthode selon l'une des revendications 1 à 10, comprenant en outre :

   /S41/ - calculer une valeur moyenne ΔTav de ΔT(k), pour k=j à j+N
   /S42/- calculer la vitesse de l'onde de pouls (VOP) définie comme VOP(k) = longueur(P) / ΔTav,
   /S5/- évaluer à partir de là la rigidité artérielle (AS) de l'utilisateur.

12. Procédé selon la revendication 11, dans lequel la taille (UH) de l'utilisateur est prise en compte à l'étape /S42/, à savoir longueur(P) = F1 (UH).

13. Méthode selon la revendication 11, dans laquelle les évaluations ultérieures de la vitesse de l'onde de pouls sont enregistrées pour un utilisateur particulier (U) afin de former une courbe d'historique, et un écart dans cette courbe est notifié à cet utilisateur particulier.

14. Système comprenant un capteur acoustique (4) configuré pour être couplé à la poitrine de l'utilisateur, un brassard (2) comportant une vessie gonflable (53) configurée pour être placée à une position prédéfinie autour d'un membre de l'utilisateur, par exemple au niveau du bras gauche (BG), une unité pneumatique comportant au moins une pompe (7) configurée pour gonfler et dégonfler la vessie gonflable, et un capteur de pression (61), un trajet sanguin artériel (P) étant défini à partir du coeur jusqu'à la position prédéfinie, le système est configuré pour exécuter la méthode comprenant une série d'étapes appelée procédure PTT :

   /A1/- acquérir des signaux acoustiques au niveau du capteur acoustique,
   /A2/- acquisition des signaux de pression sanguine au niveau du capteur de pression, image de la pression sanguine instantanée prévalant à la position prédéfinie,
   /S1/- déterminer l'instant de fermeture de la valve aortique T1(k) à partir des signaux acoustiques, l'instant de fermeture de la valve aortique T1(k) étant défini comme un deuxième son significatif (B2) du battement cardiaque,
/S2/- déterminer ensuite, à partir des signaux de circulation sanguine, un point caractéristique (M2) survenant à l'instant T2(k), le point caractéristique (M2) de la courbe du signal de pression étant défini comme une succession d'un sommet maximal (M1) et d'un sommet minimal (M2), ledit instant T2(k) étant défini comme l'instant où se produit ledit sommet minimal,
/S3/- calculer une différence de temps, définie comme ΔT(k) = T2(k) - T1(k)
/Sloop/- en répétant, pour chaque battement de coeur, les étapes /S1/ à /S3/ jusqu'à ce qu'un critère d'arrêt (SC) soit atteint.

15. Support lisible par ordinateur codé avec des instructions qui, lorsqu'elles sont exécutées par un ordinateur, entraînent l'exécution d'une méthode selon l'une des revendications 1 à 13.

FIG. 1

FIG. 2

FIG. 3

FIG. 4

FIG. 5

FIG. 6

FIG. 7

**FIG. 8**

**FIG. 9**

FIG. 10

FIG. 15

FIG. 11

Re-inflate at PT1

Start

INFLATE — DEFLATE — Determine Pulse Transit Time — TX — End

Monitor Pressure Waves

TMBP | TMPTT

ECG analysis | ECG analysis

Phonocardiogram analysis

**FIG. 12**

Start → ACQ Signal → Band Pass Filter → Assess Wave characteristics → End

Amplitude
Time position
Q factor

**FIG. 13**

T1 Determination

T2 Determination

SC true

/S0/ — /S1/ — /S2/ — /S3/ — /S4/ — /S5/ — /S6/

T1 | T2 | ΔT | PWV | Arterial Stiffness | Wireless Transmit

Repetition at each heart beat

**FIG. 14**

**EP 3 375 359 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160235325 A
- US 2003220577 A